# EUROPEAN PATENT SPECIFICATION

(11) **EP 3 187 333 B1**
(45) Date of publication and mention of the grant of the patent: **31.07.2019**
(21) Application number: 15836776.3
(22) Date of filing: 18.08.2015
(51) Int. Cl.: A41D 13/00, A41D 13/11, A61F 13/00, A61F 13/15, A61F 13/49, B32B 5/02, B23B 7/12, B32B 25/10, B32B 27/08, B32B 27/12, B32B 27/32

(54) **STRETCHABLE LAMINATE, AND ARTICLE INCLUDING SAME**
DEHNBARES LAMINAT UND ARTIKEL DAMIT
STRATIFIÉ EXTENSIBLE, ET ARTICLE CONTENANT CELUI-CI

(30) Priority: 26.08.2014 JP 2014171313
(43) Date of publication of application: 05.07.2017
(73) Proprietor: Nitto Denko Corporation, Ibaraki-shi, Osaka 567-8680 (JP)
(72) Inventor: IKISHIMA, Shinsuke, Ibaraki-shi Osaka 567-8680 (JP); UCHIDA, Shou, Ibaraki-shi Osaka 567-8680 (JP); KONDOU, Hiroyuki, Ibaraki-shi Osaka 567-8680 (JP)
(74) Representative: Müller-Boré & Partner Patentanwälte PartG mbB
(86) International application number: PCT/JP2015/073102
(87) International publication number: WO 2016/031620

(56) References cited:
- WO-A1-2004/060665
- WO-A1-2012/125344
- JP-A- 2010 075 683
- JP-A- 2011 514 178
- JP-A- 2011 514 391
- US-A1- 2005 287 892
- US-A1- 2007 249 254
- US-A1- 2011 177 735

## Description

### Technical Field

The present invention relates to a stretchable laminate and an article including the stretchable laminate.

### Background Art

Various stretchable laminates are proposed for articles such as sanitary articles, for example, diapers and masks (see, for example, Patent Literatures 1, 2 and 3).

As such materials, a stretchable laminate including an elastomer layer and a non-woven fabric layer arranged on at least one side of the elastomer layer has been proposed. In such stretchable laminate, the elastomer layer and the non-woven fabric layer are generally bonded onto each other with an adhesive or a pressure-sensitive adhesive.

However, the related-art stretchable laminate including the elastomer layer and the non-woven fabric layer arranged on at least one side of the elastomer layer involves a problem in that its oil resistance is low. For example, the stretchable laminate involves a problem in that the elastomer layer in the stretchable laminate is perforated with a hole or broken by an oil component in an adhesive or a pressure-sensitive adhesive or by an aliphatic oil, such as a baby oil.

### Citation List

### Patent Literature

[PTL 1] JP 2012-187857 A
[PTL 2] JP 3830818 B2
[PTL 3] WO 2004/060665 A1
[PTL 4] US 2011/0177735 A1

### Summary of Invention

### Technical Problem

The present invention has been made to solve the problem of the related art, and an object of the present invention is to provide a stretchable laminate excellent in oil resistance. Another object of the present invention is to provide an article including such stretchable laminate.

### Solution to Problem

A stretchable laminate according to one embodiment of the present invention includes: an elastomer layer; and a non-woven fabric layer arranged on at least one side of the elastomer layer, in which: the elastomer layer includes a plurality of layers; and at least one outer layer of the plurality of layers contains an olefin-based elastomer, as described in the present claims.

The elastomer layer includes three layers, and outer layers on both sides of the elastomer layer each contain the olefin-based elastomer.

The content of the olefin-based elastomer in the outer layer is from 90 wt% to 100 wt%.

In a preferred embodiment, the content of the olefin-based elastomer in the outer layer is from 95 wt% to 100 wt%.

The olefin-based elastomer includes an α-olefin-based elastomer.

The α-olefin-based elastomer includes a propylene-based elastomer.

In a preferred embodiment, the α-olefin-based elastomer is produced by using a metallocene catalyst.

In a preferred embodiment, an elastomer layer except the outer layer containing the olefin-based elastomer in the elastomer layer contains 50 wt% to 100 wt% of an elastomer resin.

In a preferred embodiment, the elastomer layer except the outer layer containing the olefin-based elastomer in the elastomer layer contains 80 wt% to 100 wt% of an elastomer resin.

In a preferred embodiment, the elastomer layer except the outer layer containing the olefin-based elastomer in the elastomer layer contains 95 wt% to 100 wt% of an elastomer resin.

In a preferred embodiment, the elastomer layer except the outer layer containing the olefin-based elastomer in the elastomer layer contains an elastomer resin, and the elastomer resin includes at least one kind selected from an olefin-based elastomer, a styrene-based elastomer, a vinyl chloride-based elastomer, a urethane-based elastomer, an ester-based elastomer, and an amide-based elastomer.

In a preferred embodiment, the elastomer resin includes at least one kind selected from the olefin-based elastomer and the styrene-based elastomer.

In a preferred embodiment, the elastomer layer has a thickness of from 20 µm to 200 µm.

In a preferred embodiment, the elastomer layer has a thickness of from 30 µm to 100 µm.

In a preferred embodiment, the non-woven fabric layer is formed of a non-woven fabric having a basis weight of from 10 gsm to 30 gsm.

In a preferred embodiment, the stretchable laminate further includes a hot-melt pressure-sensitive adhesive between the elastomer layer and the non-woven fabric layer.

A stretchable laminate according to one embodiment of the present invention includes: an elastomer layer; and a non-woven fabric layer arranged on at least one side of the elastomer layer, in which when the stretchable laminate is bonded and fixed onto a glass plate in a state of being extended by 100%, and 0.5 mL of a baby oil (manufactured by Pigeon Corporation, Baby Oil P, main component: caprylic/capric triglyceride) is dropped onto a surface of the stretchable laminate, the stretchable laminate is free from rupturing 10 minutes after the dropping.

An article according to one embodiment of the present invention includes the stretchable laminate according to the embodiment of the present invention.

### Brief Description of Drawings

FIG. **1** is a schematic sectional view of a stretchable laminate according to a preferred embodiment of the present invention.
FIG. **2** is a schematic view for illustrating another stretchable laminate according to a preferred embodiment of the present invention.
FIG. **3** is a schematic sectional view for illustrating one preferred embodiment of an elastomer layer.
FIG. **4** is a schematic view of a state in which a non-woven fabric is coated with a hot-melt pressure-sensitive adhesive in a striped manner in a flow direction of a production line as viewed from a top surface thereof.

### Description of Embodiments

### <<<<Stretchable Laminate>>>>

A stretchable laminate of the present invention is a stretchable laminate including an elastomer layer and a non-woven fabric layer arranged on at least one side of the elastomer layer. The stretchable laminate of the present invention may include any appropriate other layer as long as the stretchable laminate includes an elastomer layer and a non-woven fabric layer arranged on at least one side of the elastomer layer and the effects of the present invention are not impaired. The number of such any appropriate other layers may be only one, or may be two or more.

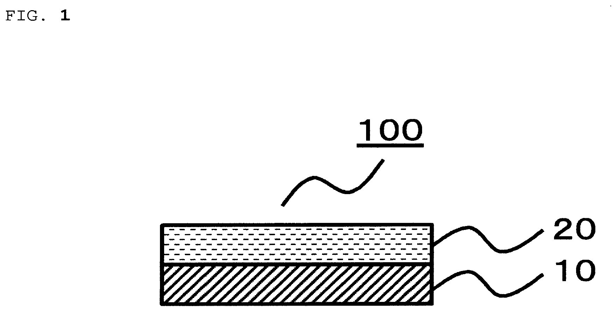

FIG. **1** is a schematic sectional view of a stretchable laminate according to a preferred embodiment of the present invention. A stretchable laminate **100** illustrated in FIG. **1** includes an elastomer layer **10** and a non-woven fabric layer **20** arranged on only one side of the elastomer layer **10.** A material for bonding the elastomer layer **10** and the non-woven fabric layer **20** may be present therebetween. Examples of such material include an adhesive, a pressure-sensitive adhesive, and a hot-melt pressure-sensitive adhesive.

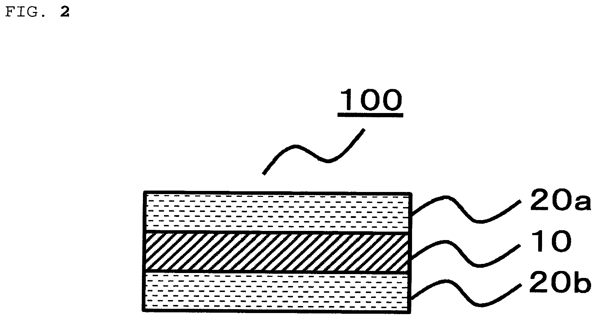

FIG. **2** is a schematic sectional view of another stretchable laminate according to a preferred embodiment of the present invention. A stretchable laminate **100** illustrated in FIG. **2** includes an elastomer layer **10,** a non-woven fabric layer **20a** arranged on one side of the elastomer layer **10,** and a non-woven fabric layer **20b** arranged on the elastomer layer **10** on an opposite side to the non-woven fabric layer **20a.** A material for bonding the elastomer layer **10** and the non-woven fabric layer **20a** and/or for bonding the elastomer layer **10** and the non-woven fabric layer **20b** may be present therebetween. Examples of such material include an adhesive, a pressure-sensitive adhesive, and a hot-melt pressure-sensitive adhesive.

The thickness of the stretchable laminate of the present invention varies depending on the thickness of the elastomer layer or the thickness of the non-woven fabric layer and is preferably from 1.0 mm to 0.1 mm, more preferably from 0.8 mm to 0.15 mm, still more preferably from 0.6 mm to 0.15 mm, particularly preferably from 0.5 mm to 0.2 mm, most preferably from 0.45 mm to 0.2 mm. When the thickness of the stretchable laminate of the present invention falls within such range, the laminate can be easily used as a material used in articles such as sanitary articles, for example, diapers and masks.

### <<Elastomer Layer>>

The elastomer layer includes a plurality of layers. Any appropriate number may be adopted as the number of such plurality of layers. The number of such plurality of layers is preferably 3.

In the elastomer layer, at least one outer layer of the plurality of layers contains an olefin-based elastomer. That is, a mode in which only one outer layer of the plurality of layers contains the olefin-based elastomer is permitted, and a mode in which both outer layers of the plurality of layers each contain the olefin-based elastomer is also permitted. When, in the elastomer layer including the plurality of layers, at least one outer layer of the plurality of layers contains the olefin-based elastomer, a stretchable laminate excellent in oil resistance can be provided.

The olefin-based elastomer may be only one kind of elastomer, or may be a blend of two or more kinds of elastomers.

In addition, when, in the elastomer layer including the plurality of layers, at least one outer layer of the plurality of layers contains the olefin-based elastomer, heat stability is improved and hence, for example, heat decomposition at the time of film formation inproducing the stretchable laminate of the present invention can be suppressed. In addition, when, in the elastomer layer including the plurality of layers, at least one outer layer of the plurality of layers contains the olefin-based elastomer, storage stability is improved and hence the fluctuation of values for physical properties during the storage of the stretchable laminate of the present invention can be suppressed.

In addition, when, in the elastomer layer including the plurality of layers, the olefin-based elastomer is adopted for at least one outer layer of the plurality of layers, steps in the production of the elastomer layer can be simplified, and hence processing cost can be reduced. This is because of the following reason: when the olefin-based elastomer is adopted, extrusion molding can be performed by using fewer kinds of resins in the production of the elastomer layer, and hence the need for the production of a master batch can be eliminated.

The content of the olefin-based elastomer in the outer layer containing the olefin-based elastomer is from 90 wt% to 100 wt%, preferably from 95 wt% to 100 wt% in terms of the expression of the effects of the present invention. When the content of the olefin-based elastomer in the outer layer containing the olefin-based elastomer falls within the range, a stretchable laminate more excellent in oil resistance can be provided.

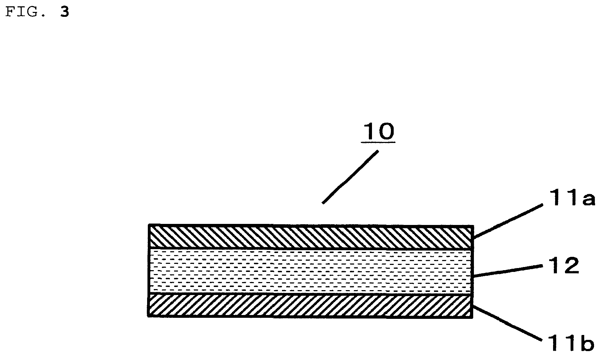

FIG. **3** is a schematic sectional view for illustrating one preferredembodimentof the elastomer layer. In FIG.**3**, the elastomer layer **10** is formed of three layers. Outer layers **11a** and **11b** on both sides of the elastomer layer each contain an olefin-based elastomer, and the elastomer layer includes an intermediate layer **12** between the outer layer **11a** and the outer layer **11b.**

Examples of the olefin-based elastomer in the outer layer include an olefin block copolymer, an olefin random copolymer, an ethylene copolymer, a propylene copolymer, an ethylene olefin block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (a-olefin) copolymer, an ethylene (a-olefin) random copolymer, an ethylene (a-olefin) block copolymer, and combinations thereof.

The olefin-based elastomer in the outer layer has a density of preferably from 0.890 g/cm³ to 0.830 g/cm³, more preferably from 0.888 g/cm³ to 0.835 g/cm³, still more preferably from 0.886 g/cm³ to 0.835 g/cm³, particularly preferably from 0.885 g/cm³ to 0.840 g/cm³, most preferably from 0.885 g/cm³ to 0.845 g/cm³. When the olefin-based elastomer whose density falls within the range described above is adopted for the outer layer, a stretchable laminate more excellent in oil resistance can be provided, and the heat stability is further improved, and hence, for example, the heat decomposition at the time of film formation in producing the stretchable laminate of the present invention can be further suppressed. In addition, the storage stability is further improved, and hence the fluctuation of values for physical properties during the storage of the stretchable laminate of the present invention can be further suppressed. Further, the steps in the production of the elastomer layer can be further simplified, and hence the processing cost can be further reduced.

The olefin-based elastomer in the outer layer has a MFR at 230°C and 2.16 kgf of preferably from 1.0 g/10 min to 25.0 g/10 min, more preferably from 2.0 g/10 min to 23.0 g/10 min, still more preferably from2 . 0 g/10 min to 21.0 g/10 min, particularlypreferably from 2.0 g/10 min to 20.0 g/10 min, most preferably from 2.0 g/10 min to 19.0 g/10 min. When the olefin-based elastomer whose MFR falls within the range described above is adopted for the outer layer, a stretchable laminate having more excellent in oil resistance can be provided, and the heat stability is further improved, and hence, for example, the heat decomposition at the time of film formation in producing the stretchable laminate of the present invention can be further suppressed. In addition, the storage stability is further improved, and hence the fluctuation of values for physical properties during the storage of the stretchable laminate of the present invention can be further suppressed. Further, the steps in the production of the elastomer layer can be further simplified, and hence the processing cost can be further reduced.

The olefin-based elastomer in the outer layer is specifically preferably an α-olefin-based elastomer. That is, the α-olefin-based elastomer is a copolymer of two or more kinds of α-olefins and has elastomer characteristics. Of such α-olefin-based elastomers, any one selected from an ethylene-based elastomer, a propylene-based elastomer, and a 1-butene-based elastomer is more preferred. When such α-olefin-based elastomer is adopted as the olefin-based elastomer, a stretchable laminate more excellent in oil resistance can be provided, and the heat stability is further improved, and hence, for example, the heat decomposition at the time of film formation in producing the stretchable laminate of the present invention can be further suppressed. In addition, the storage stability is further improved, and hence the fluctuation of values for physical properties during the storage of the stretchable laminate of the present invention can be further suppressed. Further, the steps in the production of the elastomer layer can be further simplified, and hence the processing cost can be further reduced.

The α-olefin-based elastomers each serving as the olefin-based elastomer in the outer layer, comprise a propylene-based elastomer. By adopting a propylene-based elastomer as the olefin-based elastomer, a stretchable laminate more excellent in oil resistance can be provided, and the heat stability is further improved, and hence, for example, the heat decomposition at the time of film formation in producing the stretchable laminate of the present invention can be further suppressed. In addition, the storage stability is further improved, and hence the fluctuation of values for physical properties during the storage of the stretchable laminate of the present invention can be further suppressed. Further, the steps in the production of the elastomer layer can be further simplified, and hence the processing cost can be further reduced.

Such α-olefin-based elastomer as described above is also available as a commercial product. Examples of such commercial product include some products in the "Tafmer" (trademark) series (such as Tafmer PN-3560) manufactured by Mitsui Chemicals, Inc., and some products in the "Vistamaxx" (trademark) series (such as Vistamaxx 6202 and Vistamaxx 7010) manufactured by Exxon Mobil Corporation.

The α-olefin-based elastomer serving as the olefin-based elastomer in the outer layer is preferably produced by using a metallocene catalyst. With the α-olefin-based elastomer produced by using a metallocene catalyst, a stretchable laminate more excellent in oil resistance can be provided, and the heat stability is further improved, and hence, for example, the heat decomposition at the time of film formation in producing the stretchable laminate of the present invention can be further suppressed. In addition, the storage stability is further improved, and hence the fluctuation of values for physical properties during the storage of the stretchable laminate of the present invention can be further suppressed. Further, the steps in the production of the elastomer layer can be further simplified, and hence the processing cost can be further reduced.

Any appropriate elastomer layer may be adopted as an elastomer layer except the outer layer containing the olefin-based elastomer in the elastomer layer including the plurality of layers (e.g., the intermediate layer **12** of FIG. **3**) as long as the effects of the present invention are not impaired. As an elastomer resin serving as a main component of such elastomer layer, there are given, for example, an olefin-based elastomer, a styrene-based elastomer, a vinyl chloride-based elastomer, a urethane-based elastomer, an ester-based elastomer, and an amide-based elastomer.

The content of the elastomer resin serving as a main component in the elastomer layer except the outer layer containing the olefin-based elastomer in the elastomer layer including the plurality of layers is preferably from 50 wt% to 100 wt%, more preferably from 70 wt% to 100 wt%, still more preferably from 80 wt% to 100 wt%, particularly preferably from 90 wt% to 100 wt%, most preferably from 95 wt% to 100 wt%. When the content of the elastomer resin serving as a main component in the elastomer layer except the outer layer containing the olefin-based elastomer in the elastomer layer including the plurality of layers falls within the range, the elastomer layer can express a sufficient elastomer characteristic.

The number of the elastomer layers except the outer layer containing the olefin-based elastomer in the elastomer layer including the plurality of layers may be one, or may be two or more.

The elastomer layer may contain any appropriate other component as long as the effects of the present invention are not impaired. Examples of such other component include any other polymer, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. The number of kinds of those components may be only one, or may be two or more. The content of the other component in the elastomer layer is preferably 10 wt% or less, more preferably 7 wt% or less, still more preferably 5 wt% or less, particularly preferably 2 wt% or less, most preferably 1 wt% or less.

The thickness of the elastomer layer is preferably from 200 µm to 20 µm, more preferably from 160 µm to 30 µm, still more preferably from 140 µm to 30 µm, particularly preferably from 120 µm to 30 µm, most preferably from 100 µm to 30 µm. When the thickness of the elastomer layer falls within such range, a stretchable laminate having more excellent fittability can be provided.

### <<Non-woven Fabric Layer>>

Any appropriate non-woven fabric layer may be adopted as the non-woven fabric layer as long as the effects of the present invention are not impaired. The number of kinds of non-woven fabrics constituting the non-woven fabric layer may be only one, or may be two or more.

Examples of the non-woven fabric constituting the non-woven fabric layer include a spunbonded non-woven web, a fluffy non-woven fabric (such as a non-woven fabric obtained by a thermal bonding method, a bonding joining method, or a spunlace method), a meltblown non-woven web, a spunlace non-woven web, a spunbonded meltblown spunbonded non-woven web, a spunbonded meltblown meltblown spunbonded non-woven web, an unjoined non-woven web, an electrospun non-woven web, a flashspun non-woven web (such as TYVEKTM from DuPont), and a carded non-woven fabric.

For example, the non-woven fabric constituting the non-woven fabric layer may contain fiber of polypropylene, polyethylene, polyester, polyamide, polyurethane, an elastomer, rayon, cellulose, acrylic, a copolymer thereof, or a blend thereof, or amixture thereof, or any other polyolefin.

The non-woven fabric constituting the non-woven fabric layer may contain fiber that is a homogeneous structural body, or may contain a bicomponent structural body, such as a sheath/core structure, a side-by-side structure, a sea-island structure, and any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992).

The basis amount of the non-woven fabric constituting the non-woven fabric layer is preferably 150 gsm or less, more preferably 100 gsm or less, still more preferably 50 gsm or less, particularly preferably from 10 gsm to 30 gsm.

### <<Production of Stretchable Laminate of the Present Invention>>

In the production of the stretchable laminate of the present invention, when the elastomer layer and the non-woven fabric layer are directly laminated (for example, in the cases of FIG. **1** and FIG. **2**), the elastomer layer and the non-woven fabric layer need to be bonded onto each other. Examples of such bonding method include: (1) a method involving laminating an elastomer layer formed by extrusion from a T-die of an extruder and a non-woven fabric layer separately fed from a rolled body; (2) a method involving laminating an elastomer layer and a non-woven fabric layer by co-extrusion; (3) a method involving bonding an elastomer layer and a non-woven fabric layer, which are prepared separately, with an adhesive; (4) a method involving forming a non-woven fabric layer on an elastomer layer formed by any appropriate method through use of a meltblown method or the like; and (5) thermally laminating or ultrasonically welding an elastomer layer and a non-woven fabric layer.

The elastomer layer and the non-woven fabric layer may be bonded onto each other with a hot-melt pressure-sensitive adhesive. When the hot-melt pressure-sensitive adhesive is used, the need to add a tackifier as a component of the elastomer layer is reduced, and hence, for example, the extrusion stability is improved, a problem in that the tackifier adheres to a forming roll can be suppressed, and a contamination problem of the production line by volatile matter contamination or the like caused by the tackifier can be suppressed.

When the hot-melt pressure-sensitive adhesive is used for bonding the elastomer layer and the non-woven fabric layer, in the case of applying the method (1) described above, the non-woven fabric layer separately fed from a rolled body may be coated with the hot-melt pressure-sensitive adhesive before being laminated with the elastomer layer.

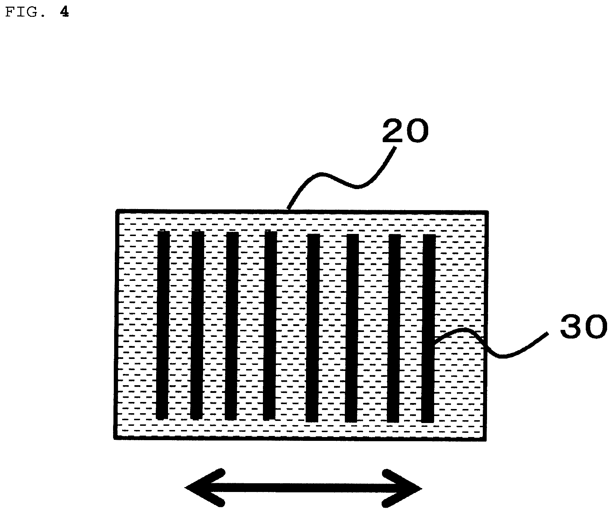

When the hot-melt pressure-sensitive adhesive is used for bonding the elastomer layer and the non-woven fabric layer, it is not necessary to coat the whole surface of the non-woven fabric layer with the hot-melt pressure-sensitive adhesive. For example, as illustrated in FIG. **4****,** the non-woven fabric layer **20** may be coated with a hot-melt pressure-sensitive adhesive **30** in a striped manner in a flow direction of the production line, or the hot-melt pressure-sensitive adhesive **30** may be applied onto the non-woven fabric layer **20** in a dotted manner. Through the coating with the hot-melt pressure-sensitive adhesive in a striped manner, portions in which the hot-melt pressure-sensitive adhesive is present and portions in which the hot-melt pressure-sensitive adhesive is absent are formed in a striped manner, and therefore, particularly in a direction perpendicular to the stripe pattern (in the direction of the arrow of FIG. **3**), the stretchability of the stretchable laminate can be further improved.

The stretchable laminate of the present invention may be subjected to treatments referred to as pre-stretching treatment and activation treatment after laminating the elastomer layer and the non-woven fabric layer. Specifically, stretching treatment is performed in a width direction of the stretchable laminate or treatment in which a fiber structure of a part of the region of the non-woven fabric layer is mechanically broken may be performed. When such treatments are performed, the stretchable laminate can be stretched by a smaller force.

### <<Application of Stretchable Laminate of the Present Invention>>

The stretchable laminate of the present invention can be used in any appropriate article in which the effects of the present invention can be effectively utilized. That is, the article of the present invention includes the stretchable laminate of the present invention. A typical example of such article is a sanitary article. Examples of such sanitary article include a diaper (in particular, an ear portion of a disposable diaper), a supporter, and a mask.

### Examples

The present invention is hereinafter specifically described by way of Examples. However, the present invention is by no means limited to these Examples. Test and evaluation methods in Examples and the like are as described below. In addition, "part (s) " means "part(s) by weight" and "%" means "wt%" unless otherwise stated.

### <Evaluation of Dropping Oil Resistance>

Each of stretchable laminates obtained in Examples and Comparative Examples was bonded and fixed onto a glass plate in a state of being extended by 100%. 0.5 mL of a baby oil (manufactured by Pigeon Corporation, Baby Oil P, main component: caprylic/capric triglyceride) was dropped onto the surface of the extended stretchable laminate. A stretchable laminate that ruptured 10 minutes after the dropping was evaluated as ×, and a stretchable laminate that did not rupture 10 minutes after the dropping was evaluated as o.

### <40°C Holding force Test>

667 parts of a propylene/1-butene copolymer (manufactured by REXtac, LLC, trade name: REXTAC RT 2788), 520 parts of a tackifier (manufactured by Kolon Industries, Inc., trade name: SUKOREZ SU-100 S), 100 parts of liquid paraffin (manufactured by Petro yag, trade name: White Oil Pharma Oyster 259), and 13 parts of an antioxidant (manufactured by BASF, trade name: Irganox 1010) were blended to provide a hot-melt pressure-sensitive adhesive. A sheet obtained by applying the hot-melt pressure-sensitive adhesive onto an OPP film (35 µm) at 15 g/m² was cut into a size of 25 mm in width. The resultant sheet was bonded onto each of the non-woven fabric surfaces (one surface side, whole surface applied portion) of the stretchable laminates produced in Examples and Comparative Examples with a width of 25 mm and a length of 15 mm and compressively bonded thereonto by two reciprocations under a load of 1 kg. The resultant was left to standstill at room temperature for 10 minutes after the compressive bonding, and then left to stand still for 30 minutes under an environment of 40°C. The resultant stretchable film was set on a holding force testing machine, and a load of 1 kg was applied to the OPP film side thereof to stretch the film. 0.1 mL of a baby oil (manufactured by Pigeon Corporation, Baby Oil P, main component: caprylic/capric triglyceride) was applied to the surface of the stretched stretchable film. A stretchable film that ruptured and fell after the application was evaluated as ×, and a time required for the falling to occur was measured. A stretchable film that did not rupture or fall even 3 hours after the application was evaluated as o.

### <Elasticity Test>

Each of the stretchable laminates obtained in Examples and Comparative Examples was cut in a CD direction so as to have a width of 30 mm. Only a stripe portion thereof was set on a tension testing machine (manufactured by Shimadzu Corporation: AG-20kNG) at a distance between chucks of 40 mm, and was extended by 100% at a tension speed of 300 mm/min. After having been extended by 100%, the laminate was fixed in an extended state and held at room temperature for 10 minutes. After a lapse of 10 minutes, the laminate was released from the extended state, and the initial distance between the chucks, i.e., 40 mm (A) and the width of the film after the test, i.e., (40+α) mm (B) were measured. After that, a fluctuation ratio was calculated from the expression "[{(B)-(A)}/(A)]×100." A stretchable laminate whose fluctuation ratio was more than 20% was evaluated as ×, and a stretchable laminate whose fluctuation ratio was less than 20% was evaluated as o.

### <Forming Conditions>

In Examples and Comparative Examples, an elastomer layer (hereinafter sometimes referred to as elastic film) was formed by extrusion molding by extruding three layers in two types (A layer/B layer/A layer) through use of a T-die molding machine. The extrusion temperatures were set under the following conditions.
A layer: 200°C
B layer: 200°C
Die temperature: 200°C

A non-woven fabric (PP carded type, basis amount=24 gsm) was bonded onto both surfaces of the elastic film extruded from the T-die through use of a roll to provide a stretchable laminate. In this case, a hot-melt pressure-sensitive adhesive was applied onto a bonded side of the non-woven fabric so as to alternately have a portion (A) in which the hot-melt pressure-sensitive adhesive had been applied onto the whole surface (7 g/m²) with a width of 30 mm and a portion (B) in which the hot-melt pressure-sensitive adhesive was applied in a striped manner (pressure-sensitive adhesive width: 1 mm, interval: 1 mm) (15 g/m²) with a width of 41 mm.

### [Example 1]

100 wt% of an olefin-based resin (manufactured by Exxon Mobil Corporation, trade name: Vistamaxx 6202) was loaded into an A layer in an extrusion machine, and a formulation of 65 wt% of an olefin-based resin (manufactured by Exxon Mobil Corporation, trade name: Vistamaxx 6202), 30 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (1) having the construction of A layer/B layer/A layer=9 µm/42 µm/9 µm in total of 60 µm.

The resultant elastic film (1) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (1) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 2]

100 wt% of an olefin-based resin (manufactured by Exxon Mobil Corporation, trade name: Vistamaxx 6202) was loaded into an A layer in an extrusion machine, anda formulation of 65 wt% of an olefin-based resin (manufactured by Exxon Mobil Corporation, trade name: Vistamaxx 6202), 30 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (2) having the construction of A layer/B layer/A layer=6.75 µm/31.5 µm/6.75 µm in total of 45 µm.

The resultant elastic film (2) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (2) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 3]

A formulation of 50 wt% of an olefin-based resin (manufactured by Exxon Mobil Corporation, trade name: Vistamaxx 6202) and 50 wt% of an olefin-based resin (manufactured by Mitsui Chemicals, Inc., trade name: Tafmer PN-3560) was loaded into an A layer in an extrusion machine, and a formulation of 45 wt% of an olefin-based resin (manufactured by Exxon Mobil Corporation, trade name: Vistamaxx 3000), 50 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (3) having the construction of A layer/B layer/A layer=6.75 µm/31.5 µm/6.75 µm in total of 45 µm.

The resultant elastic film (3) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (3) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 4]

A formulation of 50 wt% of an olefin-based resin (manufactured by Exxon Mobil Corporation, trade name: Vistamaxx 6202) and 50 wt% of an olefin-based resin (manufactured by Mitsui Chemicals, Inc., trade name: Tafmer PN-3560) was loaded into an A layer in an extrusion machine, and a formulation of 25 wt% of an olefin-based resin (manufactured by Exxon Mobil Corporation, trade name: Vistamaxx 3000), 70 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (4) having the construction of A layer/B layer/A layer=6.75 µm/31.5 µm/6.75 µm in total of 45 µm.

The resultant elastic film (4) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (4) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 5]

100 wt% of an olefin-based resin (manufactured by Exxon Mobil Corporation, trade name: Vistamaxx 7010) was loaded into an A layer in an extrusion machine, and a formulation of 45 wt% of an olefin-based resin (manufactured by Exxon Mobil Corporation, trade name: Vistamaxx 3000), 50 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (5) having the construction of A layer/B layer/A layer=6.75 µm/31.5 µm/6.75 µm in total of 45 µm.

The resultant elastic film (5) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (5) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 6]

100 wt% of an olefin-based resin (manufactured by Exxon Mobil Corporation, trade name: Vistamaxx 7010) was loaded into an A layer in an extrusion machine and a formulation of 25 wt% of an olefin-based resin (manufactured by Exxon Mobil Corporation, trade name: Vistamaxx 3000), 70 wt% of an olefin-based resin (manufactured by Mitsui Chemicals, Inc., trade name: Tafmer PN-3560), and 5 wt% of a white pigment (titanium oxide manufactured by Dupont, trade name: Ti-Pure R103) was loaded into a B layer in the extrusion machine to extrude an elastic film (6) having the construction of A layer/B layer/A layer=6.75 µm/31.5 µm/6.75 µm in total of 45 µm.

The resultant elastic film (6) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (6) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 7]

100 wt% of an olefin-based resin (manufactured by Exxon Mobil Corporation, trade name: Vistamaxx 7010) was loaded into an A layer in an extrusion machine, and a formulation of 50 wt% of a SIS-based resin (manufactured by Zeon Corporation, trade name: Quintac 3399), 45 wt% of a SBS-based resin (manufactured by Kraton Polymers, Inc. , trade name: Kraton D1191), and 5 wt% of a white pigment (titanium oxide manufactured by Dupont, trade name: Ti-Pure R103) was loaded into a B layer in the extrusion machine to extrude an elastic film (7) having the construction of A layer/B layer/A layer=6.75 µm/31.5 µm/6.75 µm in total of 45 µm.

The resultant elastic film (7) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (7) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 8]

100 wt% of an olefin-based resin (manufactured by Exxon Mobil Corporation, trade name: Vistamaxx 7010) was loaded into an A layer in an extrusion machine, and a formulation of 50 wt% of a SIS-based resin (manufactured by Zeon Corporation, trade name: Quintac 3399), 45 wt% of a SBS-based resin (manufactured by Kraton Polymers, Inc. , trade name: Kraton D1191), and 5 wt% of a white pigment (titanium oxide manufactured by Dupont, trade name: Ti-Pure R103) was loaded into a B layer in the extrusion machine to extrude an elastic film (8) having the construction of A layer/B layer/A layer=9 µm/42 µm/9 µm in total of 60 µm.

The resultant elastic film (8) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (8) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 9]

100 wt% of an olefin-based resin (manufactured by Exxon Mobil Corporation, trade name: Vistamaxx 7010) was loaded into an A layer in an extrusion machine, and a formulation of 95 wt% of a SBS-based resin (manufactured by Kraton Polymers, Inc., trade name: Kraton D1191) and 5 wt% of a white pigment (titanium oxide manufactured by Dupont, trade name: Ti-Pure R103) was loaded into a B layer in the extrusion machine to extrude an elastic film (9) having the construction of A layer/B layer/A layer=6.75 µm/31.5 µm/6.75 µm in total of 45 µm.

The resultant elastic film (9) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (9) having two portions (Al) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Example 10]

100 wt% of an olefin-based resin (manufactured by Exxon Mobil Corporation, trade name: Vistamaxx 6202) was loaded into an A layer in an extrusion machine, and a formulation of 65 wt% of the olefin-based resin (manufactured by Exxon Mobil Corporation, trade name: Vistamaxx 6202), 30 wt% of a SBS-based resin (manufactured by Kraton Polymers, Inc., trade name: Kraton 1730), and 5 wt% of a white pigment (titanium oxide manufactured by Dupont, trade name: Ti-Pure R103) was loaded into a B layer in the extrusion machine to extrude an elastic film (10) having the construction of A layer/B layer/A layer=6.75 µm/31.5 µm/6.75 µm in total of 45 µm.

The resultant elastic film (10) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (10) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1.

### [Comparative Example 1]

100 wt% of a SIS-based resin (manufactured by Zeon Corporation, trade name: Quintac 3399) was loaded into an A layer in an extrusion machine, and a formulation of 95 wt% of the SIS-based resin (manufactured by Zeon Corporation, trade name: Quintac 3399) and 5 wt% of a white pigment (titanium oxide manufactured by Dupont, trade name: Ti-Pure R103) was loaded into a B layer in the extrusion machine to extrude an elastic film (C1) having the construction of A layer/B layer/A layer=9 µm/42 µm/9 µm in total of 60 µm.

The resultant elastic film (C1) and a SIS-based hot-melt pressure-sensitive adhesive (manufacturedby Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C1) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 2]

100 wt% of a SIS-based resin (manufactured by Zeon Corporation, trade name: Quintac 3620) was loaded into an A layer in an extrusion machine, and a formulation of 95 wt% of the SIS-based resin (manufactured by Zeon Corporation, trade name: Quintac 3620) and 5 wt% of a white pigment (titanium oxide manufactured by Dupont, trade name: Ti-Pure R103) was loaded into a B layer in the extrusion machine to extrude an elastic film (C2) having the construction of A layer/B layer/A layer=9 µm/42 µm/9 µm in total of 60 µm.

The resultant elastic film (C2) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C2) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 3]

100 wt% of a SBS-based resin (manufactured by Kraton Polymers, Inc., trade name: Kraton D1191) was loaded into an A layer in an extrusion machine, and a formulation of 95 wt% of the SBS-based resin (manufactured by Kraton Polymers, Inc., trade name: Kraton D1191) and 5 wt% of a white pigment (titanium oxide manufactured by Dupont, trade name: Ti-Pure R103) was loaded into a B layer in the extrusion machine to extrude an elastic film (C3) having the construction of A layer/B layer/A layer=9 µm/42 µm/9 µm in total of 60 µm.

The resultant elastic film (C3) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C3) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 4]

100 wt% of a SBS-based resin (manufactured by Kraton Polymers, Inc., trade name: Kraton 1730) was loaded into an A layer in an extrusion machine, and a formulation of 95 wt% of the SBS-based resin (manufactured by Kraton Polymers, Inc., trade name: Kraton 1730) and 5 wt% of a white pigment (titanium oxide manufactured by Dupont, trade name: Ti-Pure R103) was loaded into a B layer in the extrusion machine to extrude an elastic film (C4) having the construction of A layer/B layer/A layer=9 µm/42 µm/9 µm in total of 60 µm.

The resultant elastic film (C4) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C4) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 5]

100 wt% of a SEBS-based resin (manufactured by Asahi Kasei Chemicals Corporation, trade name: Tuftec H1051) was loaded into an A layer in an extrusion machine, and a formulation of 95 wt% of the SEBS-based resin (manufactured by Asahi Kasei Chemicals Corporation, trade name: Tuftec H1051) and 5 wt% of a white pigment (titanium oxide manufactured by Dupont, trade name: Ti-Pure R103) was loaded into a B layer in the extrusion machine to extrude an elastic film (C5) having the construction of A layer/B layer/A layer=9 µm/42 µm/9 µm in total of 60 µm.

The resultant elastic film (C5) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C5) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 6]

100 wt% of a SBS-based resin (manufactured by Kraton Polymers, Inc., trade name: Kraton D1191) was loaded into an A layer in an extrusion machine, and a formulation of 25 wt% of a SIS-based resin (manufactured by Zeon Corporation, trade name: Quintac 3399), 70 wt% of the SBS-based resin (manufactured by Kraton Polymers, Inc. , trade name: Kraton D1191), and 5 wt% of a white pigment (titanium oxide manufactured by Dupont, trade name: Ti-Pure R103) was loaded into a B layer in the extrusion machine to extrude an elastic film (C6) having the construction of A layer/B layer/A layer=9 µm/42 µm/9 µm in total of 60 µm.

The resultant elastic film (C6) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C6) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 7]

100 wt% of a SBS-based resin (manufactured by Kraton Polymers, Inc., trade name: Kraton D1191) was loaded into an A layer in an extrusion machine, and a formulation of 50 wt% of a SIS-based resin (manufactured by Zeon Corporation, trade name: Quintac 3399), 45 wt% of the SBS-based resin (manufactured by Kraton Polymers, Inc. , trade name: Kraton D1191), and 5 wt% of a white pigment (titanium oxide manufactured by Dupont, trade name: Ti-Pure R103) was loaded into a B layer in the extrusion machine to extrude an elastic film (C7) having the construction of A layer/B layer/A layer=9 µm/42 µm/9 µm in total of 60 µm.

The resultant elastic film (C7) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C7) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 8]

100 wt% of a SBS-based resin (manufactured by Kraton Polymers , Inc., trade name: Kraton D1191) was loaded into an A layer in an extrusion machine, and a formulation of 65 wt% of an olefin-based resin (manufactured by Exxon Mobil Corporation, trade name: Vistamaxx 6202), 30 wt% of a SBS-based resin (manufactured by Kraton Polymers, Inc., trade name: Kraton 1730), and 5 wt% of a white pigment (titanium oxide manufactured by Dupont, trade name: Ti-Pure R103) was loaded into a B layer in the extrusion machine to extrude an elastic film (C8) having the construction of A layer/B layer/A layer=9 µm/42 µm/9 µm in total of 60 µm.

The resultant elastic film (C8) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C8) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 9]

100 wt% of an olefin-based resin (manufactured by Japan Polypropylene Corporation, trade name: Wintec WFX4) was loaded into an A layer in an extrusion machine, and a formulation of 95 wt% of a SBS-based resin (manufactured by Kraton Polymers, Inc., trade name: Kraton D1191) and 5 wt% of a white pigment (titanium oxide manufactured by Dupont, trade name: Ti-Pure R103) was loaded into a B layer in the extrusion machine to extrude an elastic film (C9) having the construction of A layer/B layer/A layer=9 µm/42 µm/9 µm in total of 60 µm.

The resultant elastic film (C9) and a SIS-based hot-melt pressure-sensitive adhesive (manufacturedby Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C9) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

### [Comparative Example 10]

100 wt% of an olefin-based resin (manufactured by Japan Polyethylene Corporation, trade name: Novatec LD LC720) was loaded into an A layer in an extrusion machine, and a formulation of 95 wt% of a SBS-based resin (manufactured by Kraton Polymers, Inc., trade name: Kraton D1191) and 5 wt% of a white pigment (titanium oxide manufactured by Dupont, trade name: Ti-Pure R103) was loaded into a B layer in the extrusion machine to extrude an elastic film (C10) having the construction of A layer/B layer/A layer=9 µm/42 µm/9 µm in total of 60 µm.

The resultant elastic film (C10) and a SIS-based hot-melt pressure-sensitive adhesive (manufactured by Bento Bantçιlιk, trade name: AC280) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (C10) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 2.

**Table 1**

| | | Example | | | | | | | | | |
|---|---|---|---|---|---|---|---|---|---|---|---|
| | | 1 | 2 | 3 | 4 | 5 | 6 | 7 | 8 | 9 | 10 |
| A layer resin (1) | | Vistamaxx 6202 | ← | ← | ← | Vistamaxx 7010 | Vistamaxx 7010 | Vistamaxx 7010 | Vistamaxx 7010 | Vistamaxx 7010 | Vistamaxx 6202 |
| A layer resin (2) | | - | - | Tafmer PN-3560 | ← | - | - | - | - | - | - |
| B layer resin (1) | | Vistamaxx 6202 | ← | Vistamaxx 3000 | ← | ← | ← | Quintac 3399 | Quintac 3399 | - | Vistamaxx 6202 |
| B layer resin (2) | | Tafmer PN-3560 | ← | ← | ← | ← | ← | Kraton D1191 | Kraton D1191 | Kraton D1191 | Kraton 1730 |
| B layer resin (3) | | TiO2 | ← | ← | ← | ← | ← | ← | ← | ← | ← |
| A layer formulation (1)/(2) | | 100/0 | 100/0 | 50/50 | 50/50 | 100/0 | 100/0 | 100/0 | 100/0 | 100/0 | 100/0 |
| B layer formulation (1)/(2)/(3) | | 65/30/5 | ← | 45/50/5 | 25/70/5 | 45/50/5 | 25/70/5 | 50/45/5 | 50/45/5 | 0/95/5 | 65/30/5 |
| A/B/A thickness | µm | 9/42/9 | 6.75/31.5/ 6.75 | 6.75/31.5/ 6.75 | 6.75/31.5/ 6.75 | 6.75/31.5/ 6.75 | 6.75/31.5/ 6.75 | 6.75/31.5/ 6.75 | 9/42/9 | 6.75/31.5/ 6.75 | 6.75/31.5/ 6.75 |
| Total thickness of elastic film | µm | 60 | 45 | 45 | 45 | 45 | 45 | 45 | 60 | 45 | 45 |
| Kind of hot-melt pressure-sensi tive adhesive | | AC280 | AC280 | AC280 | AC280 | AC280 | AC280 | AC280 | AC280 | AC280 | AC280 |
| Type of non-woven fabric | | Carded 24 g/m² | Carded 24 g/m² | Carded 24 g/m² | Carded 24 g/m² | Carded 24 g/m² | Carded 24 g/m² | Carded 24 g/m² | Carded 24 g/m² | Carded 24 g/m² | Carded 24 g/m² |
| | | | | | | | | | | | |
| Dropping oil resistance After 10 min | | ○ | ○ | ○ | ○ | ○ | ○ | ○ | ○ | ○ | ○ |
| 40°C holding force test Retention time | min | >180 | >180 | >180 | >180 | >180 | >180 | >180 | >180 | >180 | >180 |
| 40°C holding force test Presence or absence of film break | | ○ | ○ | ○ | ○ | ○ | ○ | ○ | ○ | ○ | ○ |
| Elasticity | | ○ | ○ | ○ | ○ | ○ | ○ | ○ | ○ | ○ | ○ |

**Table 2**

| | | Comparative Example | | | | | | | | | |
|---|---|---|---|---|---|---|---|---|---|---|---|
| | | 1 | 2 | 3 | 4 | 5 | 6 | 7 | 8 | 9 | 10 |
| A layer resin | | Quintac 3399 | Quintac 3620 | Kraton D1191 | Kraton 1730 | Tuftec H1051 | Kraton D1191 | Kraton D1191 | Kraton D1191 | Wintec WFX4 | Novatec LD LC720 |
| B layer resin (1) | | Quintac 3399 | Quintac 3620 | Kraton D1191 | Kraton 1730 | Tuftec H1051 | Quintac 3399 | Quintac 3399 | Vistamaxx 6202 | Kraton D1191 | Kraton D1191 |
| B layer resin (2) | | - | - | - | - | - | Kraton D1191 | Kraton D1191 | Kraton 1730 | - | - |
| B layer resin (3) | | TiO2 | ← | ← | ← | ← | ← | ← | ← | ← | ← |
| B layer formulation (1)/(2)/(3) | | 95/0/5 | ← | ← | ← | ← | 25/70/5 | 50/45/5 | 65/30/5 | 95/0/5 | 95/0/5 |
| A/B/A thickness | µm | 9/42/9 | 9/42/9 | 9/42/9 | 9/42/9 | 9/42/9 | 9/42/9 | 9/42/9 | 9/42/9 | 9/42/9 | 9/42/9 |
| Total thickness of elastic film | µm | 60 | 60 | 60 | 60 | 60 | 60 | 60 | 60 | 60 | 60 |
| Kind of hot-melt pressure-sensit ive adhesive | | AC280 | AC280 | AC280 | AC280 | AC280 | AC280 | AC280 | AC280 | AC280 | AC280 |
| Type of non-woven fabric | | Carded 24 g/m² | Carded 24 g/m² | Carded 24 g/m² | Carded 24 g/m² | Carded 24 g/m² | Carded 24 g/m² | Carded 24 g/m² | Carded 24 g/m² | Carded 24 g/m² | Carded 24 g/m² |
| | | | | | | | | | | | |
| Dropping oil resistance After 10 min | | × | × | × | × | × | × | × | × | ○ | ○ |
| 40°C holding force test Retention time | min | 65 | 69 | 30 | 37 | 55 | 21 | 45 | 94 | 180 | 180 |
| 40°C holding force test Presence or absence of film break | | × | × | × | × | × | × | × | × | ○ | ○ |
| Elasticity | | ○ | ○ | ○ | ○ | ○ | ○ | ○ | ○ | × | × |

### Industrial Applicability

The stretchable laminate of the present invention can be used in any appropriate article in which the effects of the present invention can be effectively utilized. That is, the article of the present invention includes the stretchable laminate of the present invention. A typical example of such article is a sanitary article. Examples of such sanitary article include a diaper (in particular, an ear portion of a disposable diaper), a supporter, and a mask.

### Reference Signs List

- **100**: stretchable laminate
- **10**: elastomer layer
- **11a**: outer layer
- **11b**: outer layer
- **12**: intermediate layer
- **20**: non-woven fabric layer
- **20a**: non-woven fabric layer
- **20b**: non-woven fabric layer
- **30**: hot-melt pressure-sensitive adhesive

## Claims

1. A stretchable laminate, including:
an elastomer layer; and
a non-woven fabric layer arranged on at least one side of the elastomer layer, wherein:
the elastomer layer comprises three layers, and outer layers on both sides of the elastomer layer each contain an olefin-based elastomer;
the content of the olefin-based elastomer in the outer layers is from 90 wt% to 100 wt%;
the olefin-based elastomer comprises an α-olefin-based elastomer; and
the α-olefin-based elastomer comprises a propylene-based elastomer.

2. The stretchable laminate according to claim 1, wherein the content of the olefin-based elastomer in the outer layers is from 95 wt% to 100 wt%.

3. The stretchable laminate according to claim 1, wherein the α-olefin-based elastomer is produced by using a metallocene catalyst.

4. The stretchable laminate according to claim 1, wherein an elastomer layer except the outer layers containing the olef in-based elastomer in the elastomer layer contains 50 wt% to 100 wt% of an elastomer resin.

5. The stretchable laminate according to claim 4, wherein the elastomer layer except the outer layers containing the olef in-based elastomer in the elastomer layer contains 80 wt% to 100 wt% of an elastomer resin.

6. The stretchable laminate according to claim 5, wherein the elastomer layer except the outer layers containing the olefin-based elastomer in the elastomer layer contains 95 wt% to 100 wt% of an elastomer resin.

7. The stretchable laminate according to claim 1, wherein an elastomer layer except the outer layers containing the olefin-based elastomer in the elastomer layer contains an elastomer resin, and the elastomer resin comprises at least one kind selected from an olefin-based elastomer, a styrene-based elastomer, a vinyl chloride-based elastomer, a urethane-based elastomer, an ester-based elastomer, and an amide-based elastomer.

8. The stretchable laminate according to claim 7, wherein the elastomer resin comprises at least one kind selected from the olefin-based elastomer and the styrene-based elastomer.

9. The stretchable laminate according to claim 1, wherein the elastomer layer has a thickness of from 20 µm to 200 µm, preferably from 30 µm to 100 µm.

10. The stretchable laminate according to claim 1, wherein the non-woven fabric layer is formed of a non-woven fabric having a basis weight of from 10 gsm to 30 gsm.

11. The stretchable laminate according to claim 1, further comprising a hot-melt pressure-sensitive adhesive between the elastomer layer and the non-woven fabric layer.

12. An article, comprising the stretchable laminate of claim 1.

## Patentansprüche

1. Dehnbares Laminat, einschließlich:
eine Elastomerschicht; und
eine Textilverbundstoffschicht, die auf mindestens einer Seite der Elastomerschicht angeordnet ist, wobei:
die Elastomerschicht drei Schichten umfasst, und äußere Schichten auf beiden Seiten der Elastomerschicht jeweils ein Olefin-basiertes Elastomer enthalten;
der Gehalt des Olefin-basierten Elastomers in den äußeren Schichten von 90 Gew.-% bis 100 Gew.-% beträgt;
das Olefin-basierte Elastomer ein α-Olefin-basiertes Elastomer umfasst; und
das α-Olefin-basierte Elastomer ein Propylen-basiertes Elastomer umfasst.

2. Dehnbares Laminat nach Anspruch 1, wobei der Gehalt des Olefin-basierten Elastomers in den äußeren Schichten von 95 Gew.-% bis 100 Gew.-% beträgt.

3. Dehnbares Laminat nach Anspruch 1, wobei das α-Olefin-basierte Elastomer unter Verwendung eines Metallocenkatalysators hergestellt ist.

4. Dehnbares Laminat nach Anspruch 1, wobei eine Elastomerschicht mit Ausnahme der äußeren Schichten, die das Olefin-basierte Elastomer in der Elastomerschicht enthalten, 50 Gew.-% bis 100 Gew.-% eines Elastomerharzes enthält.

5. Dehnbares Laminat nach Anspruch 4, wobei die Elastomerschicht mit Ausnahme der äußeren Schichten, die das Olefin-basierte Elastomer in der Elastomerschicht enthalten, 80 Gew.-% bis 100 Gew.-% eines Elastomerharzes enthält.

6. Dehnbares Laminat nach Anspruch 5, wobei die Elastomerschicht mit Ausnahme der äußeren Schichten, die das Olefin-basierte Elastomer in der Elastomerschicht enthalten, 95 Gew.-% bis 100 Gew.-% eines Elastomerharzes enthält.

7. Dehnbares Laminat nach Anspruch 1, wobei eine Elastomerschicht mit Ausnahme der äußeren Schichten, die das Olefin-basierte Elastomer in der Elastomerschicht enthalten, ein Elastomerharz enthält, und das Elastomerharz mindestens eine Art, ausgewählt aus einem Olefin-basierten Elastomer, einem Styrol-basierten Elastomer, einem Vinylchlorid-basierten Elastomer, einem Urethan-basierten Elastomer, einem Ester-basierten Elastomer und einem Amid-basierten Elastomer, umfasst.

8. Dehnbares Laminat nach Anspruch 7, wobei das Elastomerharz mindestens eine Art, ausgewählt aus dem Olefin-basierten Elastomer und dem Styrol-basierten Elastomer, umfasst.

9. Dehnbares Laminat nach Anspruch 1, wobei die Elastomerschicht eine Dicke von 20 µm bis 200 µm, vorzugsweise von 30 µm bis 100 µm, aufweist.

10. Dehnbares Laminat nach Anspruch 1, wobei die Textilverbundstoffschicht aus einem Textilverbundstoff mit einem Flächengewicht von 10 g/m² bis 30 g/m² gebildet ist.

11. Dehnbares Laminat nach Anspruch 1, weiter umfassend einen druckempfindlichen Schmelzklebstoff zwischen der Elastomerschicht und der Textilverbundstoffschicht.

12. Gegenstand, umfassend das dehnbare Laminat nach Anspruch 1.

## Revendications

1. Stratifié extensible, comprenant:
une couche d'élastomère; et
une couche de tissu non tissé disposée sur au moins un côté de la couche d'élastomère, dans lequel:
la couche d'élastomère comprend trois couches, et des couches extérieures sur les deux côtés de la couche d'élastomère contiennent chacune un élastomère à base d'oléfine;
la teneur en élastomère à base d'oléfine dans les couches extérieures va de 90% en poids à 100% en poids;
l'élastomère à base d'oléfine comprend un élastomère à base d'α-oléfine; et
l'élastomère à base d'α-oléfine comprend un élastomère à base de propylène.

2. Stratifié extensible selon la revendication 1, dans lequel la teneur en élastomère à base d'oléfine dans les couches extérieures va de 95% en poids à 100% en poids.

3. Stratifié extensible selon la revendication 1, dans lequel l'élastomère à base d'a-oléfine est produit en utilisant un catalyseur métallocène.

4. Stratifié extensible selon la revendication 1, dans lequel une couche d'élastomère, à l'exception des couches extérieures contenant l'élastomère à base d'oléfine dans la couche d'élastomère, contient de 50% en poids à 100% en poids d'une résine d'élastomère.

5. Stratifié extensible selon la revendication 4, dans lequel la couche d'élastomère, à l'exception des couches extérieures contenant l'élastomère à base d'oléfine dans la couche d'élastomère, contient de 80% en poids à 100% en poids d'une résine d'élastomère.

6. Stratifié extensible selon la revendication 5, dans lequel la couche d'élastomère, à l'exception des couches extérieures contenant l'élastomère à base d'oléfine dans la couche d'élastomère, contient de 95% en poids à 100% en poids d'une résine d'élastomère.

7. Stratifié extensible selon la revendication 1, dans lequel une couche d'élastomère, à l'exception des couches extérieures contenant l'élastomère à base d'oléfine dans la couche d'élastomère, contient une résine d'élastomère et la résine d'élastomère comprend au moins une sorte sélectionnée parmi un élastomère à base d'oléfine, un élastomère à base de styrène, un élastomère à base de chlorure de vinyle, un élastomère à base d'uréthane, un élastomère à base d'ester et un élastomère à base d'amide.

8. Stratifié extensible selon la revendication 7, dans lequel la résine d'élastomère comprend au moins une sorte sélectionnée parmi l'élastomère à base d'oléfine et l'élastomère à base de styrène.

9. Stratifié extensible selon la revendication 1, dans lequel la couche d'élastomère présente une épaisseur allant de 20 µm à 200 µm, de préférence de 30 µm à 100 µm.

10. Stratifié extensible selon la revendication 1, dans lequel la couche de tissu non tissé est formée d'un tissu non tissé ayant un poids de base allant de 10 gsm à 30 gsm.

11. Stratifié extensible selon la revendication 1, comprenant en outre un adhésif thermofusible sensible à la pression entre la couche d'élastomère et la couche de tissu non tissé.

12. Article comprenant le stratifié extensible selon la revendication 1.
